# EUROPÄISCHE PATENTSCHRIFT

(11) **EP 1 198 263 B1**
(45) Veröffentlichungstag und Bekanntmachung des Hinweises auf die Patenterteilung: **16.04.2003**
(21) Anmeldenummer: 00951086.8
(22) Anmeldetag: 27.07.2000
(51) Int. Cl.: A61M 5/20

(54) **Vorrichtung zum automatischen Injizieren von Injektionsflüssigkeiten**
Device for automatically injecting injection liquids
Dispositif d'injection automatique de liquides à injecter

(30) Priorität: 27.07.1999 AT 50999 U
(43) Veröffentlichungstag der Anmeldung: 24.04.2002
(73) Patentinhaber: Pharma Consult Ges.M.B.H., A-1210 Wien (AT)
(72) Erfinder: PICKHARD, Ewald, A-2203 Grossebersdorf (AT)
(74) Vertreter: Haffner, Thomas M.
(86) Internationale Anmeldenummer: AT0000207
(87) Internationale Veröffentlichungsnummer: WO01007104

(56) Entgegenhaltungen:
- EP-A- 0 154 593
- EP-A- 0 695 554
- DE-A- 19 532 410
- US-A- 5 658 259
- US-A- 5 695 472
- US-A- 5 709 668

## Beschreibung

Die Erfindung betrifft eine Vorrichtung zum automatischen Injizieren von Injektionsflüssigkeiten mit einem in axialer Richtung unterteilten Gehäuse, dessen Teile lösbar miteinander verbindbar sind, wobei in einem ersten Gehäuseteil ein axial verschieblicher Druckbolzen geführt ist, welcher gegen einen Kraftspeicher einschiebbar und in der eingeschobenen Lage verriegelbar ist und unter Entlastung des Kraftspeichers ausfahrbar ist und in einem zweiten Gehäuseteil eine in einer Kanülenführung festsitzende Injektionskanüle und eine Ampulle in axialer Richtung verschiebbar gelagert sind, wobei Injektionskanüle relativ zur Ampulle in axialer Richtung verschiebbar gelagert ist und an ihrer der Ampulle zugewandten Seite als Durchstichstück für die Ampulle ausgebildet ist. Dadurch, daß nun die Injektionskanüle an ihrer der Ampulle zugewandten Seite als Durchstichstück für die Ampulle ausgebildet ist und daß die Injektionskanüle relativ zur Ampulle in axialer Richtung verschiebbar gelagert ist, kann die Ampulle im Inneren des zweiten Gehäuseteiles, des Injektors, gelagert werden, ohne fest mit der Injektionskanüle verbunden zu sein. Die Injektionsflüssigkeit kann so über Jahre hinweg sicher in der Ampulle verwahrt werden, wobei die Ampulle mit keinen anderen Bauteilen fest verbunden ist und die Haltbarkeit der Injektionsflüssigkeit nicht negativ beeinflußt wird. Durch die Verwendung einer Ampulle wird außerdem die Herstellung des Injektorteils wesentlich vereinfacht und weiters der Vorteil erreicht, daß aus einem größeren Angebot zur Verfügung stehender unterschiedlicher Injektionsflüssigkeiten gewählt werden kann als dies bei Fertigspritzen der Fall ist.

Erst im Falle der Anwendung wird die Ampulle unter der Kraft des durch Entlastung des Kraftspeichers ausfahrenden Druckbolzens in Richtung der Injektionskanüle verschoben und von der Injektionskanüle, welche an ihrer der Ampulle zugewandten Seite als Durchstichstück ausgebildet ist, durchstochen. Dadurch wird ein Verlegen der Injektionskanüle mit Sicherheit verhindert und sichergestellt, daß die Injektionsflüssigkeit ungehindert aus der Ampulle austreten kann, nachdem die Injektionskanüle aus dem Injektorgehäuse ausgeschoben worden und ins Gewebe eingedrungen ist.

Eine Einrichtung der eingangs genannten Art ist in der US-A-5 709 668 beschrieben und ist unter der Bezeichnung Autoinjektor bekannt geworden. Bei den bekannten Einrichtungen handelt es sich um füllfedergroße Instrumente, welche bei Eintreten einer Notsituation das Injizieren eines Notfallmittels in den Körper erleichtern. Autoinjektoren werden beispielsweise bei Allergienotfällen, z.B. bei Insektenstichen, Schlangenbissen usw., angewendet aber auch im Militärbereich um beispielsweise Vergiftungen durch C-Kampfstoffe rasch entgegenzuwirken. Die bekannten Einrichtungen sind meist als Einwegeinrichtungen konzipiert und werden daher nach einmaliger Verwendung entsorgt.

Aus der AT 303 251 ist eine weitere Injektionsvorrichtung bekannt geworden, welche aus zwei ineinander verschraubbaren Gehäuseteilen, den einen federbelastbaren Druckbolzen enthaltenden Aktivator und den die Ampulle und die Injektionsnadel als untrennbare Einheit enthaltenden Injektor, besteht. Nach Entriegeln des federbelasteten Druckbolzens übt dieser eine Kraft auf den Kolbenstopfen der Ampulle aus, worauf zunächst die Ampulle gemeinsam mit der Injektionsnadel innerhalb des Injektorgehäuses in axialer Richtung verschoben wird, sodaß die Injektionsnadel in die Körperoberfläche eindringt und in der Folge die in der Ampulle befindliche Flüssigkeit unter einen so hohen Druck gelangt, daß eine zwischen Ampulle und Injektionsnadel vorhandene Abdichtmembran bricht und dadurch die Flüssigkeit ausgestoßen wird. Nachteilig an dieser bekannten Ausbildung ist jedoch, daß die aufgebrochene Membran die Injektionsnadel verlegen kann, wodurch ein rasches Ausstoßen der Injektionsflüssigkeit verhindert wird. Weiters ist bei dieser Ausbildung die Entsorgung des Autoinjektors nach dem Gebrauch problematisch, da die Injektionsnadel aus dem Gehäuse herausragt, was ein hohes Verletzungs- bzw. Infektionsrisiko mit sich bringt.

Die vorbekannten Autoinjektoren weisen weiters den Nachteil auf, daß ein sicheres Lagern und Transportieren von Reserveampullen nicht möglich ist. Die Ampulle kann nur im Werk in den Autoinjektor eingesetzt werden. Bei den bekannten Autoinjektoren muß jeweils ein weiterer gesamter Autoinjektor als Reserveautoinjektor mitgeführt werden.

Die Erfindung zielt nun darauf ab, eine Vorrichtung eingangs genannter Art zu schaffen, welche ohne Verletzungs- bzw. Infektionsrisiko entsorgt werden kann und bei der zusätzlich die Entsorgung unter Minimierung der Anzahl der zu entsorgenden Teile möglichst umweltschonend möglich ist. Gleichzeitig zielt die Erfindung darauf ab einen Lager- und Transportbehälter für Ampullen zu schaffen, in dem die befüllte Glasampulle über Jahre hinweg sicher geschützt und dicht verschlossen gelagert werden kann. Insbesondere soll auch das Mitführen von mehreren Ampullen mit unterschiedlichen Injektionsflüssigkeiten und/oder von Reserveampullen unter Minimierung der mitzuführenden Teile ermöglicht werden. Die Lösung dieser Aufgabe erfolgt bei einer erfindungsgemäßen Vorrichtung mit den im kennzeichen des Anspruchs 1 angegebenen Merkmalen.

Dadurch, daß der zweite Gehäuseteil an seinem dem ersten Gehäuseteil zugewandten offenen Ende durch eine Verschlußkappe verschließbar ist, wird gleichzeitig ein Lager- und Transportbehälter für die Glasampulle und ein Entsorgungsbehälter für die gebrauchte Injektionskanüle und die leere Ampulle geschaffen. Der die Ampulle und die Kanüle umschließende Gehäuseteil kann somit getrennt vom Aktivatorteil aufbewahrt und transportiert werden, wobei die Ampulle sowie die Kanüle durch die Verschlußkappe über Jahre hinweg bruchsicher geschützt und bakteriendicht verschlossen sind. Dies erleichtert vor allem das Mitführen mehrerer verschiedener Ampullen. Je nach Bedarf kann der getrennt mitführbare Aktivatorteil mit dem die entsprechende Injektionsflüssigkeit enthaltenden Injektorteil verbunden werden. Das Mitführen mehrerer Aktivatorteile ist nicht notwendig, da der Aktivator wiederverwendbar ist.

Der die Kanüle umschließende Gehäuseteil eignet sich auch dadurch zur Verwendung als Entsorgungsbehälter, daß die die Kanülenführung federnd gelagert ist. Nach Gebrauch des Autoinjektors wird der Injektorteil vom Aktivator gelöst, wobei die gebrauchte, aus dem Injektorgehäuse hervorragende Injektionskanüle durch die Wirkung eines federnden Bauteils von selbst wieder in das Innere des Gehäuses gelangt. Die Ausbildung ist hierbei so getroffen sein, daß im Inneren des zweiten Gehäuseteils zwischen dem die Durchtrittsöffnung für die Injektionskanüle aufweisenden Gehäuseende und der Kanülenführung eine Schraubenfeder angeordnet ist. Dadurch, daß mit Vorteil der zweite Gehäuseteil an seinem die Durchtrittsöffnung für die Injektionskanüle aufweisenden Ende durch eine elastische Dichtscheibe verschlossen ist, bleibt das kanülenseitige Ende des Injektorgehäuses immer dicht, sodaß das Aufstecken einer Verschlußkappe nicht erforderlich ist.

Jedenfalls wird der Injektor an seinem dem ersten Gehäuseteil, dem Aktivator, zugewandten offenen Ende durch eine Verschlußkappe verschlossen, wodurch ein Entsorgungsbehälter geschaffen wird, der die gebrauchte Injektionskanüle und die leere Ampulle sicher und dichtend aufnimmt. Eine Verletzung bzw. Infektion durch die entsorgten Teile wird somit ausgeschlossen und es wird weiters die Anzahl der wegzuwerfenden Teile minimiert. Der Aktivator kann in der Folge wiederverwendet werden, indem der Druckbolzen gegen einen Kraftspeicher eingeschoben und in der eingeschobenen Lage verriegelt wird und der Aktivator mit einem neuen Injektor verbunden wird. Ein neuer, nicht verwendeter Injektor ist hierbei vor dem ersten Gebrauch immer mit einer Verschlußkappe verschlossen, wodurch ein Lager- und Transportbehälter für die Glasampulle geschaffen wird, bei welchem die Sterilität der Ampulle und der Kanüleneinheit gesichert ist. So können beispielsweise mehrere Injektoren mit unterschiedlichen Injektionsflüssigkeiten mitgeführt werden und im Notfall der passende Injektor nach Loslösen der Verschlußkappe mit dem Aktivator verbunden werden.

Wie bereits erwähnt ist die erfindungsgemäße Vorrichtung so ausgebildet, daß die das Durchstichstück für die Ampulle tragende Injektionskanüle mit einer Kanülenführung relativ zur Ampulle axial verschieblich gelagert ist. Dadurch wird sichergestellt, daß bei Krafteinwirkung des Druckbolzens auf den Kolben der Ampulle zuerst die Injektionskanüle, welche mit einer Kanülenführung axial verschieblich gelagert ist, in axialer Richtung verschoben wird und an der gewünschten Stelle durch die Haut ins Gewebe eindringt und erst danach das Durchstichstück der Injektionskanüle die Ampulle durchbricht, wobei die Kanülenführung an ihrem Mantel wenigstens ein einwärts springendes federndes Auflagestück für die Ampulle und weiters wenigstens ein auswärts springendes, federndes und mit einem entsprechend Anschlag des zweiten Gehäuseteiles zusammenwirkendes Auflagestück aufweist. Das einwärts springende federnde Auflagestück ist so dimensioniert, daß es der vom Druckbolzen ausgehenden Kraft mehr Widerstand entgegensetzt als das auswärts springende federnde Auflagestück. Dadurch dringt die Injektionskanüle unter Überwindung der Kraft des sich in Richtung zur Ampulle zurückbiegenden auswärts springenden federnden Auflagestückes zuerst in das Körperinnere ein, worauf das einwärts springende federnde Auflagestück während des Eindringens des Durchstichstückes in die Ampulle nach innen in die Kanülenführung geschoben wird. Dadurch, daß das auswärts springende federnde Auflagestück in der Transportund Lagerposition mit einem entsprechenden Anschlag des zweiten Gehäuseteiles zusammenwirkt, kann die Kanülenführung genau positioniert und in ihrer Lage gesichert im Gehäuse aufgenommen werden.

Die erfindungsgemäße Vorrichtung ist weiters so ausgebildet, daß das ein am offenen Ende des zweiten Gehäuseteiles angeordnete Gewinde bzw. Bajonett mit einem Gegengewinde bzw. Gegenbajonett des ersten Gehäuseteiles verschraubbar bzw. verrastbar ist. Dadurch kann das am zweiten Gehäuseteil angeordnete Gewinde einerseits mit einer Verschlußkappe und andererseits nach Abnahme der Verschlußkappe mit dem Aktivator verschraubt werden. Entsprechend trägt die Verschlußkappe ein Gegengewinde bzw. Gegenbajonett, welches mit einem am offenen Ende des zweiten Gehäuseteiles angeordneten Gewinde bzw. Bajonett verschraubbar bzw. verrastbar ist.

Um ein Herausfallen der Ampulle aus dem zweiten Gehäuseteil nach dem Trennen vom Aktivatorteil mit Sicherheit zu verhindern, ist mir Vorteil die Ausbildung so getroffen, daß in einem mit vergrößertem Durchmesser ausgebildeten Bereich des zweiten Gehäuseteiles ein einen Anschlag für die Ampulle aufweisender Teil verrastbar ist, welcher die Ampulle gegen Herausfallen sichert. Dadurch ist sichergestellt, daß weder die kontaminierte Injektionskanüle noch die jetzt leere Ampulle durch die Kraft der auf die Kanülenführung wirkenden, expandierenden Druckfeder aus dem zweiten Gehäuseteil herausfallen bzw. herausgeschleudert werden kann.

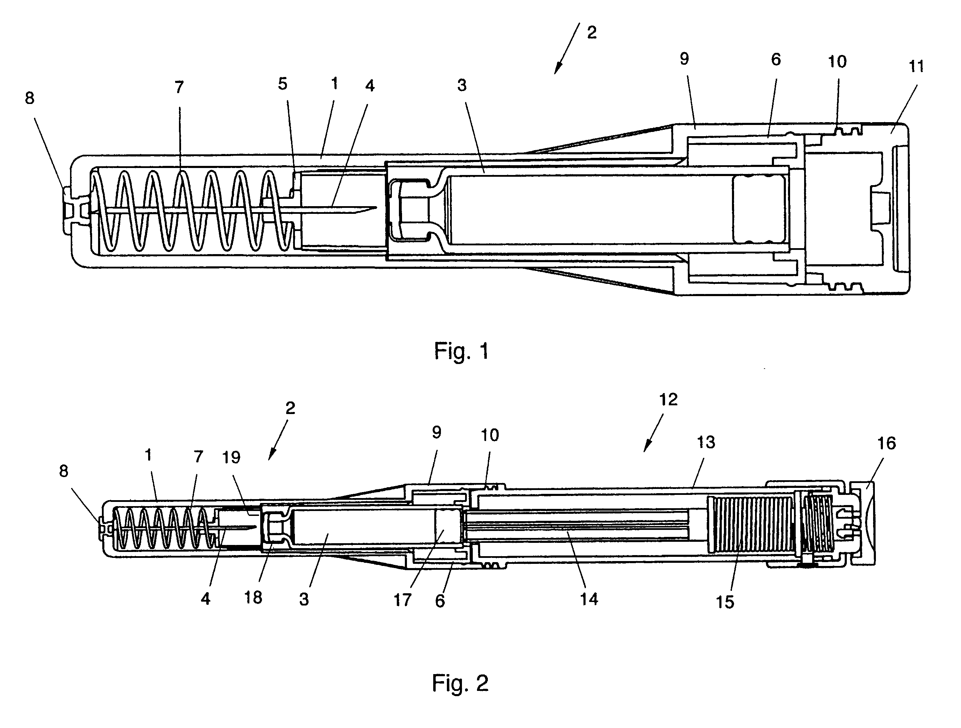

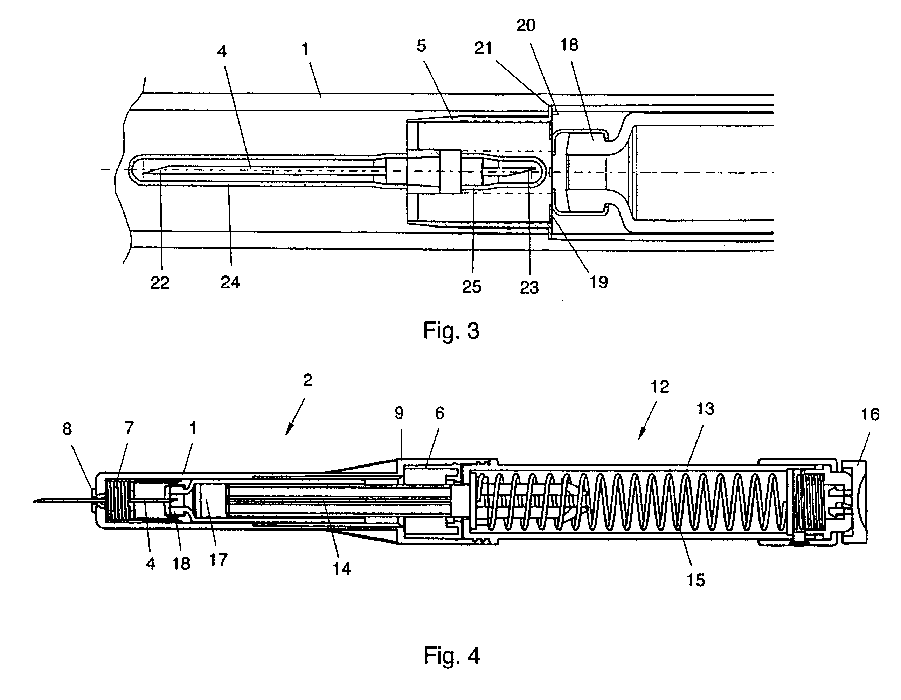

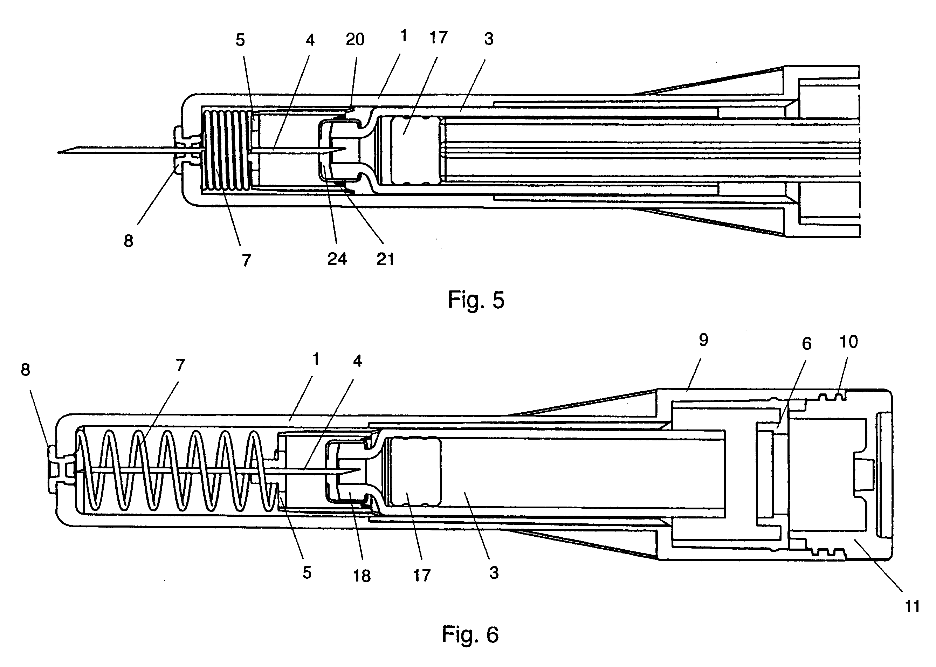

Die Erfindung wird nachfolgend anhand eines in der Zeichnung schematisch dargestellten Ausführungsbeispieles näher erläutert. In dieser zeigen Fig. 1 den Injektorteil der erfindungsgemäßen Vorrichtung mit aufgeschraubter Verschlußkappe, Fig. 2 den Injektorteil der erfindungsgemäßen Vorrichtung mit aufgeschraubtem Aktivatorteil vor Ausspritzen der Injektionsflüssigkeit, Fig. 3 eine Detailansicht der Fig. 2, Fig. 4 eine erfindungsgemäße Vorrichtung gemäß Fig. 2 nach dem Ausspritzen der Injektionsflüssigkeit, Fig. 5 eine Detailansicht der Fig. 4 und Fig. 6 den Injektorteil der erfindungsgemäßen Vorrichtung mit aufgeschraubter Verschlußkappe zur Verwendung als Entsorgungsbehälter.

In Fig. 1 ist mit 1 das Gehäuse des Injektorteils 2 der erfindungsgemäßen Vorrichtung dargestellt. In dem Gehäuse 1 sind eine Ampulle 3 und eine die Injektionskanüle 4 tragende Kanülenführung 5 axial verschieblich gelagert, wobei ein einen Anschlag für die Ampulle 3 aufweisender Teil 6 vorgesehen ist, welcher die Ampulle 3 gegen Herausfallen sichert. Weiters ist innerhalb des Gehäuses 1 eine Schraubenfeder 7 angeordnet, deren Funktion weiter unten beschrieben wird. Das Gehäuse 1 ist an seinem kanülenseitigen Ende durch eine elastische Dichtscheibe 8 verschlossen. Im Bereich 9 weist das Gehäuse 1 einen größeren Durchmesser sowie ein Gewinde 10 auf, sodaß eine Verschlußkappe 11 aufgeschraubt werden kann, wodurch nun ein Lager- und Transportbehälter für die gefüllte Glasampulle 3 geschaffen wird.

In Fig. 2 ist der Injektorteil 2 mit dem Aktivatorteil 12 verschraubt. Der Aktivatorteil 12 besteht aus einem Gehäuse 13, in welchem ein Druckbolzen 14 axial verschiebbar entgegen der Kraft einer Feder 15 verschieblich geführt ist. Die Druckfeder 15 ist bei der Darstellung nach Fig. 2 gespannt und der Druckbolzen 14 ist in der aufgezogenen Position des Druckbolzens verriegelt. Zum Auslösen ist ein Betätigungsglied 16 am hinteren Ende des Aktivatorteiles 12 vorgesehen. Der Druckbolzen 14 liegt auf dem Ampullenkolben 17 auf, wobei die Ampulle 3 ihrerseits im Bereich des abgesetzten Halses 18 auf dem federnden Auflagestück 19 der Kanülenführung 5 aufliegt. Wie sich insbesondere aus Fig. 3 ergibt, ist die Kanülenführung 5 mit einem auswärts springenden Auflagestück 20 ausgebildet, welches mit einem Anschlag 21 des Gehäuses 1 so zusammenwirkt, daß die Kanülenführung 5 im Inneren des Gehäuses 1 genau positioniert und in ihrer Lage sicher aufgenommen wird. In Fig. 3 ist weiters zu erkennen, daß die Kanüle 4 an ihrem vorderen Ende als Injektionsnadel 22 und an ihrem ampullenseitigen Ende als Durchstichstück 23 ausgebildet ist. Die Injektionskanüle 4 ist in der Darstellung gemäß Fig. 3 mittels Kanülenschutzkappen 24 und 25 hermetisch abgeschlossen. Derartige Kanülenschutzkappen können alternativ verwendet werden, wobei dann auf die elastischer Dichtscheibe (8) verzichtet wird.

Nach einer Entriegelung und einem axialen Hub des Betätigungsgliedes 16 wird der Arbeitshub des Druckbolzens 14 freigegeben. Der Druckbolzen 14 bewegt sich unter Entspannung der Feder 15 in axialer Richtung nach vorne und bewirkt unter Zwischenschaltung des Ampullenkolbens 17, welcher innerhalb der verschlossenen Ampulle 3 in axialer Richtung noch nicht beweglich ist und weiters unter Zwischenschaltung des federnden Auflagestückes 19 eine axiale Verschiebung der Kanülenführung 5 so, daß die Kanüle 4 aus dem Gehäuse 1 austaucht, wobei gleichzeitig das auswärts springende federnde Auflagestück 20 in Richtung zur Ampulle 3 zurückgebogen wird. Die elastische Dichtscheibe 8 wird dabei von der Injektionsnadel 22 durchstochen, wobei die Feder 7 zwischen dem Gehäuse 1 und der Kanülenführung 5 zusammengepreßt wird. Nach Austritt der Injektionskanüle 4 aus dem Gehäuse 1 ist die weitere axiale Verschiebung der Kanülenführung 5 nicht mehr möglich, sodaß nun die vom Druckbolzen 14 auf den Ampullenkolben 17 ausgeübte Kraft ein Verbiegen des federnden Auflagestückes 19 bewirkt und sich die Ampulle 3 nun relativ zur Kanülenführung 5 bewegen kann. Dabei durchstößt das als Durchstichstück ausgebildete ampullenseitige Ende der Injektionskanüle 4 die Ampullendichtung 24 der Ampulle 3, sodaß nun die Injektionsflüssigkeit durch die von dem Druckbolzen 14 bewirkte axiale Verschiebung des Ampullenkolbens 17 ausgepreßt wird.

In Fig. 4 ist die erfindungsgemäße Vorrichtung gemäß Fig. 2 nochmals dargestellt, wobei nun die Ampulle 3 völlig entleert ist und insbesondere aus Fig. 5 ersichtlich ist, wie die der Kraft des Druckbolzens 14 unterschiedlich große Widerstandskräfte entgegensetzenden Auflagestücke 19 und 20 verbogen werden. Die Feder 7 ist hier zusammengedrückt.

In Fig. 6 ist der nach dem Gebrauch vom Aktivatorteil 12 abgeschraubte Injektorteil 2 dargestellt, wobei die Injektionskanüle 4 durch die auf die Kanülenführung 5 wirkende Druckfeder 7 in das Gehäuse 1 zurückgeschoben wurde. Durch die elastische Dichtscheibe 8 ist das Gehäuse 1 kanülenseitig wieder dicht verschlossen, sodaß die kontaminierte Injektionsnadel geschützt im Injektorteil 2 liegt. Die Feder 7 verhindert zusätzlich eine Bewegung der Nadel derart, daß die elastische Dichtscheibe 8 nicht erneut durchdrungen wird. Der im Gehäuse angeordnete, am Teil 6 angebrachte Anschlag für die Ampulle verhindert das Herausfallen sowie das Herausnehmen der Ampulle 3. Gleichzeitig ist die Verschlußkappe 11 in das Gewinde 10 eingeschraubt, sodaß auch das andere Ende dicht verschlossen werden kann. Insgesamt ist damit eine optimale Entsorgung und Materialtrennung gewährleistet, wobei gleichzeitig eine Verletzungsgefahr durch die gebrauchte Injektionsnadel ausgeschlossen wird.

## Patentansprüche

1. Vorrichtung zum automatischen Injizieren von Injektionsflüssigkeiten mit einem in axialer Richtung unterteilten Gehäuse, dessen Teile lösbar miteinander verbindbar sind, wobei in einem ersten Gehäuseteil (13) ein axial verschieblicher Druckbolzen (14) geführt ist, welcher gegen einen Kraftspeicher einschiebbar und in der eingeschobenen Lage verriegelbar ist und unter Entlastung des Kraftspeichers (15) ausfahrbar ist und in einem zweiten Gehäuseteil (1) eine in einer Kanülenführung (5) festsitzende Injektionskanüle (4) and eine Ampulle (3) in axialer Richtung verschiebbar gelagert sind, wobei die Injektionskanüle (4) relativ zur Ampulle (3) in axialer Richtung verschiebbar gelagert ist und an ihrer der Ampulle (3) zugewandten Seite als Durchstichstück (23) für die Ampulle (3) ausgebildet ist, **dadurch gekennzeichnet, daß** im Inneren des zweiten Gehäuseteils (1) zwischen dem die Durchtrittsöffnung für die Injektionskanüle (4) aufweisenden Gehäuseende und der Kanülenführung (5) eine Schraubenfeder (7) angeordnet ist, daß die Kanülenführung (5) an ihrem Mantel wenigstens ein einwärts springendes, in axialer Richtung federndes Auflagestück (19) für die Ampulle (3) aufweist und die Kanülenführung (5) an ihrem Mantel wenigstens ein auswärts springendes federndes Auflagestück (20) aufweist, welches mit einem entsprechenden Anschlag (21) des zweiten Gehäuseteiles (1) zusammenwirkt, und daß der zweite Gehäuseteil (1) an seinem dem ersten Gehäuseteil (13) zugewandten offenen Ende ein Gewinde (10) bzw. Bajonett trägt, welches mit einem Gegengewinde bzw. Gegenbajonett des ersten Gehäuseteils (13) oder einer Verschlußkappe (11) verschraubbar bzw. verrastbar ist.

2. Vorrichtung nach Anspruch 1, **dadurch gekennzeichnet, daß** der zweite Gehäuseteil (1) an seinem die Durchtrittsöffnung für die Injektionskanüle (4) aufweisenden Ende durch eine elastische Dichtscheibe (8) verschlossen ist.

3. Vorrichtung nach Anspruch 1 oder 2, **dadurch gekennzeichnet, daß** in einem mit vergrößertem Durchmesser ausgebildeten Bereich (9) des zweiten Gehäuseteiles (1) ein einen Anschlag für die Ampulle (3) aufweisender Teil (6) verrastbar ist, welcher die Ampulle (3) gegen Herausfallen sichert.

## Claims

1. A device for automatically injecting injection liquids, including an axially divided housing whose parts are detachably connected with each other, wherein an axially displaceable pressure pin (14) is guided within a first housing part (13), which pressure pin is insertable against an energy accumulator (15) and lockable in the inserted position and extendable upon relief of the energy accumulator (15), and wherein an injection needle (4) firmly fixed in a needle guide (5) and an ampoule (3) are mounted within a second housing part in an axially displaceable manner, said injection needle (4) being mounted so as to be displaceable in the axial direction relative to the ampoule (3) and designed as a perforation piece (23) for the ampoule (3) on its side facing the ampoule (3), **characterized in that** a helical spring (7) is arranged in the interior of the second housing part (1) between the housing end comprising the passage opening of the injection needle (4) and the needle guide (5), that the needle guide (5) on its jacket comprises at least one inwardly projecting and axially resilient bearing piece (19) for the ampoule (3) and the needle guide (5) on its jacket comprises at least one outwardly projecting resilient bearing piece (20) cooperating with a mating stop (21) of the second housing part (1), and that the second housing part (1) on its open end facing the first housing part (13) carries a thread (10) or bayonet capable of being screwed or locked with a counter thread or counter bayonet, respectively, of the first housing part (13), or a closing cap (11).

2. A device according to claim 1, **characterized in that** the second housing part (1) on its end comprising the passage opening for the injection needle (4) is closed by an elastic sealing disc (8).

3. A device according to claim 1 or 2, **characterized in that** a part (6) comprising a stop for the ampoule (3) and securing the ampoule (3) against falling out is lockable in an enlarged-diameter portion (9) of the second housing part (1).

## Revendications

1. Dispositif d'injection automatique de liquides à injecter comprenant un corps partagé dans le sens axial, dont les parties peuvent être assemblées entre elles de manière amovible, une première partie de corps (13) contenant un piston de pression (14) coulissant dans le sens axial, qui peut, d'une part, être inséré en direction d'un accumulateur d'énergie (15) et verrouillé dans la position insérée et d'autre part être retiré en déchargeant l'accumulateur d'énergie (15), et une deuxième partie de corps (1) contenant une canule d'injection (4) fixe dans un guide de canule (5) et une ampoule (3) supportées de façon coulissante dans le sens axial, la canule d'injection (4) étant supportée de façon coulissante dans le sens axial par rapport à l'ampoule (3) et étant conformée de son côté orienté vers l'ampoule (3) comme un perforateur (23) pour l'ampoule (3), **caractérisé en ce qu'**un ressort hélicoïdal (7) est prévu à l'intérieur de la deuxième partie de corps (1) entre l'extrémité du corps comportant l'ouverture de passage de la canule d'injection (4) et le guide de canule (5), **en ce que** le guide de canule (5) présente sur son enveloppe au moins un élément d'appui (19) faisant ressort vers l'intérieur et élastique dans le sens axial pour l'ampoule (3) et le guide de canule (5) présente sur son enveloppe au moins un élément d'appui (20) faisant ressort vers l'extérieur et élastique qui coopère avec une butée (21) correspondante de la deuxième partie de corps (1), et **en ce que** la deuxième partie de corps (1) présente à son extrémité ouverte orientée vers la première partie de corps (13) un filetage (10) ou une baïonnette pouvant être vissé ou emboîtée avec un deuxième élément de filetage ou de baïonnette sur la première partie de corps (13) ou avec un capuchon d'obturation (11).

2. Dispositif selon la revendication 1, **caractérisé en ce que** la deuxième partie de corps (1) est fermée par un disque d'étanchéité élastique (8) à son extrémité comportant l'ouverture de passage de la canule d'injection (4).

3. Dispositif selon la revendication 1 ou 2, **caractérisé en ce qu'**une partie (6) présentant une butée pour l'ampoule (3), qui empêche l'ampoule (3) de tomber, peut être emboîtée dans une partie de diamètre agrandi (9) de la deuxième partie de corps (1).
